# FASCICULE DE BREVET EUROPEEN

(11) **EP 1 401 325 B1**
(45) Date de publication et mention de la délivrance du brevet: **25.07.2007**
(21) Numéro de dépôt: 02738239.9
(22) Date de dépôt: 13.05.2002
(51) Int. Cl.: A61B 1/267, A61B 1/24, A61B 5/103

(54) **EMBOUT DESTINE A UN DISPOSITIF D'EVALUATION DE LA SENSIBILITE DU PHARYNX ET DISPOSITIF LE COMPORTANT**
MUNDSTÜCK ZUR VERWENDUNG MIT EINEM GERÄT ZUR BESTIMMUNG DER SENSIBILITÄT DES PHARYNX UND ES ENTHALTENDES GERÄT
MOUTHPIECE INTENDED FOR A DEVICE USED TO ASSESS THE SENSITIVITY OF THE PHARYNX AND A DEVICE COMPRISING SAME

(30) Priorité: 15.05.2001 FR 0106389
(43) Date de publication de la demande: 31.03.2004
(73) Titulaire: UNIVERSITE JOSEPH FOURIER, 38041 Grenoble Cédex 9 (FR)
(72) Inventeur: DEMATTEIS, Maurice, F-38240 Meylan (FR); PEPIN, Jean-Louis, F-38000 Grenoble (FR); LEVY, Patrick, F-38000 Grenoble (FR)
(74) Mandataire: Jouvray, Marie-Andrée
(86) Numéro de dépôt international: PCT/FR2002/001595
(87) Numéro de publication internationale: WO 2002/091916

(56) Documents cités:
- WO-A-96/11627
- US-A- 2 756 742
- US-A- 3 976 054
- AVIV JONATHAN: "clinical assessment of pharyngolaryngeal sensitivity" AMERICAN JOURNAL OF MEDICINE, vol. 108, no. 4a, 6 mars 2000 (2000-03-06), pages 68s-72s, XP008000776

## Description

### Domaine technique de l'invention

L'invention concerne un embout destiné à un dispositif d'évaluation de la sensibilité du pharynx, ainsi qu'un dispositif le comportant.

### État de la technique

Il existe actuellement peu de moyens pour évaluer la sensibilité du pharynx. Or, cette évaluation est souhaitable dans un certain nombre de pathologies, qui impliquent un dysfonctionnement du pharynx, telles que les apnées du sommeil. Les procédés basés sur une stimulation mécanique ou électrique du pharynx, s'accompagnent d'effets indésirables, comme l'induction d'un réflexe nauséeux, ce qui en limite l'utilisation chez un certain nombre de sujets.

Le document WO-A-9611627 décrit un dispositif utilisant une stimulation du pharynx par une séquence de jets d'air pulsé, de durée prédéterminée, par exemple 50ms, et de pression variable, par exemple entre 0 et 10mm de mercure, par paliers de 7,5x10⁻²mm de mercure. Les jets d'air pulsé sont amenés sur la zone à tester par un tuyau flexible de faible diamètre fixé à un fibroscope qui est introduit dans le nez du patient à examiner, comme ceux couramment utilisés pour l'observation du pharynx. Le tuyau véhiculant l'air peut éventuellement être intégré au fibroscope. La réponse du patient au stimulus constitué par un jet d'air pulsé peut correspondre soit à une indication donnée par le patient, soit à l'observation d'un réflexe comme la fermeture des cordes vocales observée au moyen d'un fibroscope. Un tel dispositif est complexe et coûteux. Il nécessite en effet un plateau technique sophistiqué et des compétences spécialisées de l'utilisateur, ce qui limite le nombre de sujets pouvant être examinés.

Le document US-A-3 976 054 décrit un dispositif apte à fournir une ligne de vue directe vers le pharynx comprenant un embout buccal et un tube de guidage.

### Objet de l'invention

L'invention a pour but de remédier aux inconvénients des systèmes connus et de fournir des moyens simples, peu coûteux et limitant les effets indésirables chez le patient, pour évaluer la sensibilité du pharynx.

Selon l'invention, ce but est atteint par un embout destiné à un dispositif d'évaluation de la sensibilité du pharynx qui comporte un embout buccal ouvert et ayant des dimensions internes permettant une inspection visuelle du pharynx, un mince tube de guidage fixé le long d'une paroi interne de l'embout buccal et comportant une première extrémité articulée faisant saillie à une première extrémité de l'embout buccal, un tuyau, destiné à être connecté par une première extrémité à une source de gaz comprimé et introduit par une seconde extrémité à l'intérieur du tube de guidage, et des moyens de fixation du tuyau dans le tube de guidage dans une position prédéterminée.

L'invention concerne également un dispositif d'évaluation de la sensibilité du pharynx, comportant une source de gaz comprimé, des moyens pour insuffler du gaz en direction du pharynx, un embout selon l'invention et des moyens de mesure du débit d'un flux de gaz injecté dans la première extrémité du tuyau.

Un procédé d'utilisation d'un dispositif selon l'invention pour l'évaluation de la sensibilité du pharynx comporte l'introduction de la première extrémité de l'embout buccal dans la bouche d'un sujet, l'introduction de la seconde extrémité du tuyau dans le tube de guidage, sous contrôle visuel à travers l'embout buccal, jusqu'à ce qu'il touche le palais du sujet, le retrait du tuyau sur une distance prédéterminée mesurée par des graduations du tuyau, la connexion de la première extrémité du tuyau à la source d'air comprimé, l'injection d'un flux d'air de débit prédéterminé, variable, dans le tuyau, la sensibilité du pharynx étant déterminée en fonction de la valeur des débits pour lesquels le sujet ressent le flux d'air.

### Description sommaire des dessins

D'autres avantages et caractéristiques ressortiront plus clairement de la description qui va suivre de modes particuliers de réalisation de l'invention donnés à titre d'exemples non limitatifs, et représentés aux dessins annexés, dans lesquels :
- La figure 1 représente, en coupe transversale, un mode de réalisation particulier d'un embout selon l'invention.
- La figure 2 représente l'embout selon la figure 1 en position dans la bouche d'un patient.
- La figure 3 représente un mode particulier de réalisation d'un dispositif selon l'invention.

### Description de modes particuliers de réalisation.

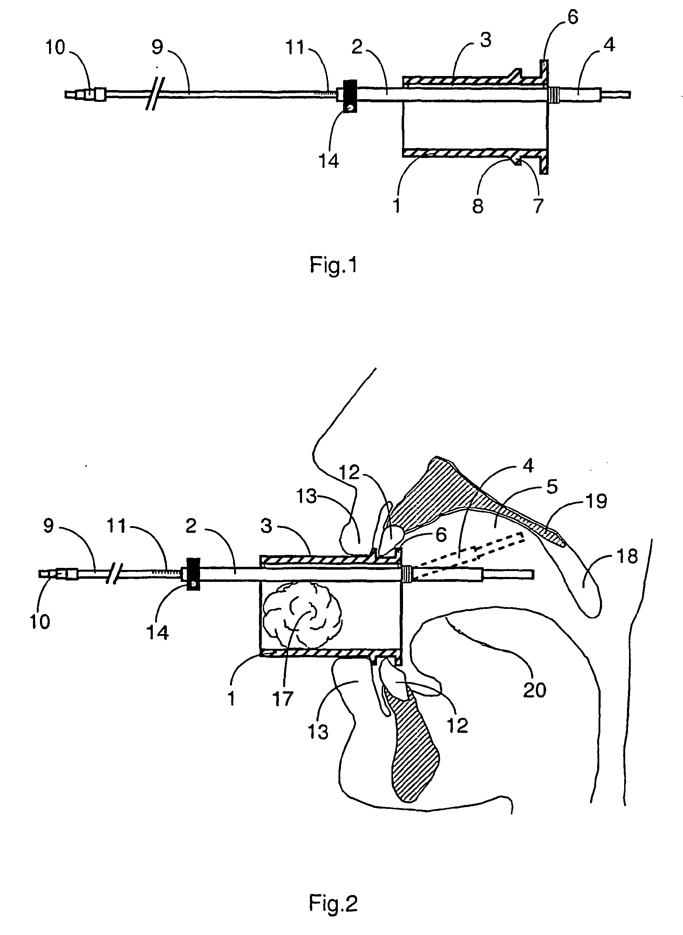

L'embout représenté aux figures 1 et 2 comporte un embout buccal 1 ouvert, constituant le corps de l'embout et constitué de préférence par un cylindre ouvert à ses deux extrémités, de quelques centimètres de diamètre et de quelques centimètres de longueur. À titre d'exemple, le cylindre peut avoir 2 à 3cm de diamètre et 4cm de long. Il peut être plus étroit pour permettre d'évaluer la sensibilité du pharynx de sujets, comme les enfants, dont l'ouverture buccale est étroite. Sa longueur peut être réduite pour rendre plus facile son maintien dans la bouche d'un sujet.

Un mince tube de guidage 2 est fixé, par exemple par de la colle 3, le long d'une paroi interne de l'embout buccal 1. Le tube de guidage 2 est, de préférence constitué par un chalumeau, c'est-à-dire un petit tube ou paille, en matière plastique, généralement utilisé pour aspirer un liquide. Il a un diamètre de quelques millimètres, par exemple 4 à 5mm, et dépasse de quelques centimètres, 3cm par exemple, de part et d'autre de l'embout buccal. Il est, de plus, articulé à une première extrémité 4 destinée à être introduite à l'intérieur de la cavité buccale 5 du sujet. Cette articulation permet de positionner l'extrémité 4 du tube de guidage 2, ainsi que le tuyau 9 décrit ci-dessous, en regard du palais membraneux 18, entre le palais osseux 19 et la langue 20, quelque soit la morphologie de la cavité orale du sujet examiné. Sur la figure 2, une seconde position de l'extrémité 4 est illustrée en pointillé.

L'embout buccal 1 comporte un rebord de maintien 6, externe, à une première extrémité destinée à être introduite dans la bouche d'un sujet. Une nervure 7, sensiblement parallèle au rebord de maintien 6, peut être prévue pour faciliter le maintien de l'embout dans la bouche du sujet. La nervure 7 comporte, de préférence, une face inclinée 8 du côté opposé au rebord de maintien 6.

L'embout comporte également un tuyau 9, destiné à être connecté par une première extrémité à une source de gaz comprimé et introduit par une seconde extrémité à l'intérieur du tube de guidage 2. Le diamètre du tuyau 9 est légèrement inférieur à celui du tube de guidage 2, de manière à pouvoir être introduit facilement dans celui-ci. Dans un mode de réalisation préféré, le diamètre du tuyau 9 est de l'ordre de 2mm. Le tuyau 9 peut, par exemple, être constitué par une sonde à oxygène de type classique, en matière plastique souple, comportant à sa première extrémité un élément 10 de raccordement à la source de gaz comprimé.

Le tuyau 9 comporte des graduations 11 destinées à coopérer avec la seconde extrémité du tube de guidage 2, opposée à sa première extrémité articulée 4, et disposée, pendant l'utilisation de l'embout, hors de la cavité buccale 5 du sujet.

Pour évaluer la sensibilité du pharynx d'un sujet, l'embout buccal 1, muni du tube de guidage 2, dont l'extrémité articulée 4 est orientée de manière appropriée, est introduit, par sa première extrémité comportant le rebord de maintien 6, dans la bouche du sujet. Comme représenté à la figure 2, les dents 12 des maxillaires supérieur et inférieur du sujet viennent se placer derrière le rebord de maintien 6, entre le rebord de maintien et la nervure 7, assurant ainsi le maintien de l'embout buccal 1 dans la bouche. Les lèvres 13 du sujet épousent alors la forme de l'embout buccal 1 et reposent notamment sur la face inclinée 8 de la nervure 7. Seule une petite partie de l'embout buccal 1 est alors dans la bouche du sujet, la majorité de l'embout buccal 1, de la nervure 7 à la seconde extrémité de l'embout buccal 1, opposée au rebord de maintien 6, faisant saillie à l'extérieur.

Les dimensions internes de l'embout buccal 1 sont telles qu'elles permettent, malgré la présence du tube de guidage 2, une inspection visuelle du pharynx. La seconde extrémité du tuyau 9 est introduite dans le tube de guidage 2, sous contrôle visuel à travers l'embout buccal 1, jusqu'à ce qu'elle touche le palais du sujet. La graduation disposée face à la seconde extrémité du tube de guidage étant notée, le tuyau est alors retiré sur une distance prédéterminée, par exemple 1 cm, vers l'extérieur. Il n'y a plus, alors de contact entre l'extrémité du tuyau et le palais, mais la distance entre cette extrémité et le palais est toujours la même, quelle que soit la morphologie du palais du sujet examiné. Une pince 14, ou à défaut du ruban adhésif, est alors utilisée pour fixer le tuyau 9, dans cette position, par rapport au tube de guidage 2.

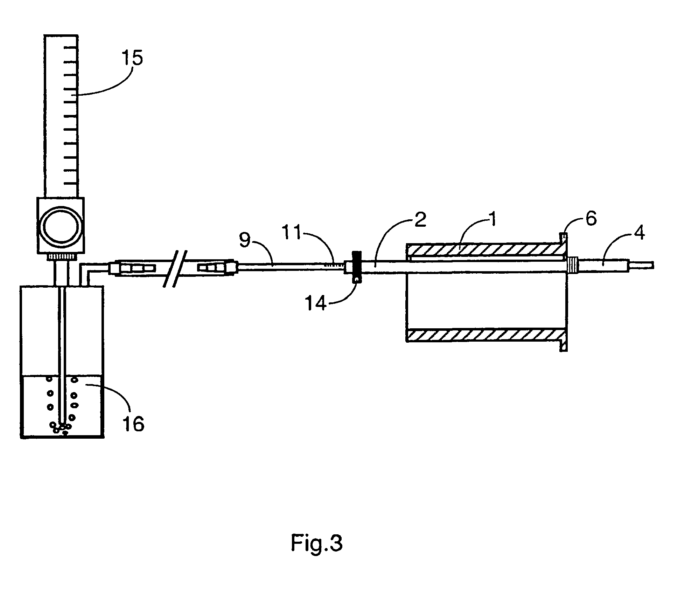

Le tuyau 9 est connecté, éventuellement par l'intermédiaire d'une connexion de plus gros diamètre et d'une prise murale, à une source de gaz comprimé, de préférence à une bouteille d'air comprimé ou à une bouteille d'oxygène. Un débitmètre 15 (figure 3) permet de régler et de mesurer avec précision le débit de gaz injecté dans le tuyau 9. Un flux de gaz, de débit prédéterminé, variable, est injecté dans le tuyau 9 et atteint la muqueuse pharyngée du palais située en regard du tuyau 9. Pour donner un premier repère au sujet examiné sur la sensation qu'il doit percevoir, un débit élevé, par exemple de 2 Umin, est administré puis est progressivement diminué par paliers, en demandant à chaque palier si le sujet perçoit encore la sensation. Il signale, selon la consigne préalablement définie avant l'examen, la perception ou la non-perception du flux gazeux. Le débit le plus faible encore perçu correspond au seuil de perception sensitive. Pour valider la mesure, la procédure est répétée au moins deux autres fois, les différentes valeurs obtenues sont alors moyennées. Le seuil de perception est ensuite mesuré en administrant des débits croissants par palier, en partant d'un débit nul. À chaque palier, le sujet est interrogé. La valeur la plus faible du flux gazeux perçu correspond au seuil de perception sensitive. La mesure est répétée au moins deux fois, les différentes valeurs obtenues sont alors moyennées. Le fait de demander à chaque palier si le sujet perçoit une sensation peut potentiellement influencer la réponse. Par conséquent, une autre façon de procéder est de demander au sujet de signaler par une consigne gestuelle, ou par tout autre moyen, lorsque la sensation pharyngée disparaît ou apparaît. Au cours de l'expérience, il est demandé au sujet de fermer les yeux et un casque anti-bruit peut être placé sur les oreilles afin de faciliter la concentration sur la perception du flux au niveau de la muqueuse pharyngée. Le casque anti-bruit évite aussi d'influencer les réponses du sujet par le son des modifications de débits gazeux. Enfin, au cours de l'examen, le sujet doit respirer par le nez afin d'éviter toute interférence entre le flux gazeux administré sur la muqueuse et l'air inspiré. À cet effet, l'embout buccal peut être obstrué par sa deuxième extrémité, notamment à l'aide d'une boule de coton 17.

Ce dispositif est très simple et peu coûteux. Les graduations du tuyau 9 suffisent, en combinaison avec un contact préalable temporaire, sous contrôle visuel, de la seconde extrémité du tuyau avec le palais, à assurer un positionnement standard pour tous les sujets. L'articulation de la première extrémité du tube de guidage 2 permet, de plus, d'adapter le dispositif à la morphologie du palais d'un grand nombre de sujets. Seul le débit du flux de gaz est pris en compte, ni sa pression, ni la durée de l'injection n'ayant à être pris en compte. Un tel dispositif peut facilement être utilisé par tout médecin disposant d'une source d'air ou d'oxygène comprimé.

Pour éviter l'assèchement de la muqueuse pharyngée au cours de l'examen, le dispositif peut comporter un humidificateur 16 (figure 3), interposé entre la source de gaz et l'entrée du tuyau 9, qui humidifie le gaz injecté dans le tuyau.

L'humidificateur 16 peut comporter de l'eau, qui peut être portée à une température prédéterminée, variable. Il est alors possible d'évaluer également la sensibilité thermique de la muqueuse pharyngée.

La méthode peut être rendue plus sensible par l'utilisation d'un anesthésique local, par exemple de la xylocaïne, pulvérisé sur la muqueuse pharyngée située en regard du tuyau 9, sous contrôle visuel à travers l'embout buccal 1 ou directement après retrait du dispositif. La sensibilité est alors réévaluée après un délai minimal permettant à l'anesthésique de produire son effet, par exemple 5 minutes. Ensuite, d'autres pulvérisations de l'anesthésique local peuvent être administrées sur la muqueuse pharyngée selon la même procédure afin de définir une relation effet-dose.

En raison de l'hétérogénéité de l'innervation sensitive de la muqueuse pharyngée, la sensibilité peut être évaluée selon la même procédure que celle décrite précédemment, sur des zones autres que celle du palais, comme par exemple le pilier des amygdales, la paroi postérieure du pharynx ou encore le nasopharynx.

En raison de l'hétérogénéité des récepteurs chimiques (chémorécepteurs) de la muqueuse pharyngée, le flux gazeux peut consister en un flux d'air, d'oxygène ou de dioxyde de carbone afin d'évaluer la part respective des différents types de récepteurs dans la perception sensitive de la muqueuse pharyngée et leur implication dans les pathologies sous-tendues par un dysfonctionnement de cette perception.

Afin d'évaluer les différentes parties de l'arc réflexe dilatateur du pharynx (partie afférente sensitive et partie efférente motrice), on peut envisager de coupler l'évaluation sensitive, telle qu'elle a été décrite précédemment, à l'évaluation de la réponse motrice, comme l'appréciation de l'activité électromyographique d'un muscle dilatateur du pharynx, par exemple le muscle génioglosse, notamment en réponse à une pression négative soumise dans les voies aériennes supérieures.

Afin de supprimer le caractère subjectif des réponses du sujet, ou encore la non-compréhension des consignes ou un défaut de coopération, on peut envisager de coupler l'évaluation de la sensibilité à l'enregistrement de potentiels évoqués somesthésiques. Il convient alors de fixer un débit d'air suffisamment élevé pour déclencher une stimulation sensitive, par exemple 2Umin, et d'administrer de manière brève et intermittente ce flux gazeux, à une fréquence donnée et pour une durée suffisante telle qu'elle permette d'évoquer une réponse corticale électro-encéphalographique (potentiel évoqué) pouvant être moyennée.

## Revendications

1. Embout destiné à un dispositif d'évaluation de la sensibilité du pharynx, comportant un embout buccal (1), ouvert et ayant des dimensions internes permettant une inspection visuelle du pharynx, et un mince tube fixé le long d'une paroi interne de l'embout buccal (1), **caractérisé en ce que** le tube étant un tube de guidage (2) comportant une première extrémité articulée (3) faisant saillie à une première extrémité de l'embout buccal (1), l'embout comporte un tuyau (9) destiné à être connecté par une première extrémité à une source de gaz comprimé et introduit par une seconde extrémité à l'intérieur du tube de guidage (2), et des moyens (14) de fixation du tuyau dans le tube de guidage dans une position prédéterminée.

2. Embout selon la revendication 1, **caractérisé en ce que** le tuyau (9) comporte des graduations (11).

3. Embout selon l'une des revendications 1 et 2, **caractérisé en ce que** l'embout buccal (1) est cylindrique.

4. Embout selon l'une quelconque des revendications 1 à 3, **caractérisé en ce que** l'embout buccal (1) comporte un rebord de maintien (6), externe, à sa première extrémité.

5. Embout selon la revendication 4, **caractérisé en ce que** l'embout buccal (1) comporte une nervure de maintien (7).

6. Dispositif d'évaluation de la sensibilité du pharynx, comportant une source de gaz comprimé et des moyens pour insuffler du gaz en direction du pharynx, **caractérisé en ce qu'**il comporte un embout selon l'une quelconque des revendications précédentes et des moyens (15) de mesure du débit d'un flux de gaz injecté dans la première extrémité du tuyau (9).

7. Dispositif selon la revendication 6, **caractérisé en ce que** le gaz est de l'air.

8. Dispositif selon la revendication 6, **caractérisé en ce que** le gaz est de l'oxygène.

9. Dispositif selon la revendication 6, **caractérisé en ce que** le gaz est du dioxyde de carbone.

10. Dispositif selon l'une quelconque des revendications 6 à 9, **caractérisé en ce qu'**il comporte des moyens humidificateurs (16) pour humidifier l'air injecté dans le tuyau.

11. Dispositif selon la revendication 10, **caractérisé en ce que** les moyens humidificateurs (16) comportent de l'eau portée à une température prédéterminée, variable.

## Claims

1. A mouthpiece intended for a device designed for evaluating the sensitivity of the pharynx, comprising an open mouthpiece (1) having internal dimensions enabling visual inspection of the pharynx and a thin guide tube (2) fixed along an internal wall of the mouthpiece (1), **characterized in that** the tube being a guide tube (2) comprising a first articulated end (3) salient from a first end of the mouthpiece (1), the mouthpiece comprises a pipe (9) designed to be connected via a first end to a compressed gas source and inserted via a second end inside the guide tube (2) and means (14) for fixing the pipe in the guide tube in a predetermined position.

2. Mouthpiece according to claim 1, **characterized in that** the pipe (9) comprises a measurement scale (11).

3. Mouthpiece according to one of the claims 1 and 2, **characterized in that** the mouthpiece (1) is cylindrical.

4. Mouthpiece according to any one of the claims 1 to 3, **characterized in that** the mouthpiece (1) comprises an external holding rim (6) at its first end.

5. Mouthpiece according to claim 4, **characterized in that** the mouthpiece (1) comprises a holding rib (7).

6. Device for evaluating the sensitivity of the pharynx, comprising a compressed gas source and means for blowing gas in the direction of the pharynx, **characterized in that** it comprises a mouthpiece according to any one of the foregoing claims and means (15) for measuring the flow rate of a gas injected into the first end of the pipe (9).

7. Device according to claim 6, **characterized in that** the gas is air.

8. Device according to claim 6, **characterized in that** the gas is oxygen.

9. Device according to claim 6, **characterized in that** the gas is carbon dioxide.

10. Device according to any one of the claims 6 to 9, **characterized in that** it comprises humidifying means (16) to humidify the air Injected Into the pipe.

11. Device according to claim 10, **characterized in that** the humidifying means (16) comprise water heated to a variable preset temperature.

## Patentansprüche

1. Ansatzstück zur Verwendung mit einem Gerät zur Bestimmung der Empfindlichkeit des Pharynx, das ein Mundstück (1), das offen ist und Innenabmessungen aufweist, die eine visuelle Untersuchung des Pharynx ermöglichen, sowie ein dünnes Rohr umfasst, das entlang einer Innenwand der Mundstücks (1) angebracht ist, **dadurch gekennzeichnet, dass**, nachdem das Rohr ein Führungsrohr (2) ist, das ein erstes angelenktes Ende (3), das an einem ersten Ende des Mundstücks (1) hervorsteht, umfasst, das Ansatzstück eine Röhre (9) umfasst, die an einem ersten Ende an eine Druckgasquelle angeschlossen und an einem zweiten Ende in das Führungsrohr (2) eingeführt werden soll, sowie Mittel (14) zur Befestigung der Röhre in dem Führungsrohr in einer vorbestimmten Position.

2. Ansatzstück nach Anspruch 1, **dadurch gekennzeichnet, dass** die Röhre (9) eine Gradeinteilung (11) umfasst.

3. Ansatzstück nach einem der Ansprüche 1 und 2, **dadurch gekennzeichnet, dass** das Mundstück (1) zylindrisch ist.

4. Ansatzstück nach einem der Ansprüche 1 bis 3, **dadurch gekennzeichnet, dass** das Mundstück (1) an seinem ersten Ende einen externen Halterand (6) aufweist.

5. Ansatzstück nach Anspruch 4, **dadurch gekennzeichnet, dass** das Mundstück (1) eine Halterippe (7) umfasst.

6. Gerät zur Bestimmung der Empfindlichkeit des Pharynx, das eine Druckgasquelle und Mittel zum Blasen von Gas zum Pharynx hin umfasst, **dadurch gekennzeichnet, dass** es ein Ansatzstück nach einem der vorstehenden Ansprüche und Mittel (15) zum Messen des Durchsatzes eines Gasstroms umfasst, der in das erste Ende der Röhre (9) eingeblasen wird.

7. Gerät nach Anspruch 6, **dadurch gekennzeichnet, dass** das Gas Luft ist.

8. Gerät nach Anspruch 6, **dadurch gekennzeichnet, dass** das Gas Sauerstoff ist.

9. Gerät nach Anspruch 6, **dadurch gekennzeichnet, dass** das Gas Kohlendioxid ist.

10. Gerät nach einem der Ansprüche 6 bis 9, **dadurch gekennzeichnet, dass** es Befeuchtungsmittel (16) zum Befeuchten der in die Röhre eingeblasenen Luft umfasst.

11. Gerät nach Anspruch 10, **dadurch gekennzeichnet, dass** die Befeuchtungsmittel (16) Wasser umfassen, das auf eine vorbestimmte, variable Temperatur gebracht wurde.
